# EUROPEAN PATENT APPLICATION

(11) **EP 1 669 079 A1**
(43) Date of publication of application: **14.06.2006**
(21) Application number: 04029195.7
(22) Date of filing: 09.12.2004
(51) Int. Cl.: A61K 33/04, A61K 33/24, A61K 31/137, A61P 3/10

(54) **Composition of SSAO substrates and metal compounds of the Vla Vlb groups of the periodic table**

(71) Applicant: Genmedica Therapeutics, Barcelona 08010 (ES)
(72) Inventor: Albericio, Fernando, 08007 Barcelona (ES); Zorzano, Antonio, 08028 Barcelona (ES); Marti, Luc, 08024 Barcelona (ES); Royo, Miriam, 08001 Barcelona (ES); Yraola, Francesc, St. Antoni de Calonge (ES)
(74) Representative: Graf von Stosch, Andreas

(57) **Abstract**

This invention relates to (pharmaceutical) compositions comprising a combination of SSAO substrates and pharmaceutically acceptable compounds of the VIa and VIb group of the periodic table, i.e. selenium (VI)-, molybdenum (VI)- and tungsten (V)/(VI) compounds, etc. and to their use as insulin mimetics, i.e., to treat illness Diabetes mellitus, Obesity, or their symptoms. A here discovered synergism between the compounds of the Vla and Vlb groups of the periodic table, i.e. selenium (VI)-, molybdenum (VI)- and tungsten (V)/(VI) compounds, etc. and the SSAO substrates makes the effective concentration of the compounds of the VIa and VIb groups of the periodic table in the (pharmaceutical) composition one order of magnitude lower than the corresponding compound of the VIa and VIb groups alone. Consequently the inventive (pharmaceutical) composition has much lower toxicity than the known compounds or compositions in the art alone, what is a crucial advantage of the former for its use in the treatment and/or prevention of Diabetes mellitus, particularly of Diabetes type 2, or Obesity.

## Description

This invention relates to (pharmaceutical) compositions comprising SSAO substrates and pharmaceutically acceptable metal salts of the Vla and Vlb groups (6^{th} main- or subgroup) of the periodic table, i.e. selenium (Vl)-, molybdenum (Vl)- and tungsten (V)/(VI) compounds, etc. and to their use as insulin mimetics, i.e., to treat Diabetes mellitus and/or its symptoms. The invention further relates to kits containing these metal salts and SSAO substrates.

The most common form of diabetes is diabetes mellitus. Diabetes mellitus is a major global health problem which is recognised by the World Health Organisation to be reaching epidemic proportions. It is now the fourth leading cause of death in most developed countries and a disease that is increasing rapidly in countries undergoing industrialisation. Diabetes mellitus is a disease caused by defective carbohydrate metabolism. It is characterized by abnormally elevated glucose levels in the plasma and urine, by excessive urine excretion and by episodic ketoacidosis. Additional symptoms of diabetes mellitus include excessive thirst, glucosuria, polyuria, lipidemia and hunger. If left untreated the disease can lead to fatal ketoacidosis. Diabetes mellitus can eventually damage the eyes, kidneys, heart, and limbs, and can endanger pregnancy. Criteria, which clinically establish an individual as suffering from diabetes mellitus, include fasting plasma glucose levels in excess of 126mg/dL (7mmol/L). Normal levels are typically less than 100mg/dL (<5.6mmol/L). Alternatively, patients may show a plasma glucose levels in excess of 200mg/dL (11 mmol/L) at two times points during a glucose tolerance test (GTT), one of which must be within 2 hrs of ingestion of glucose.

Diabetes mellitus is usually classified into two major types, type 1 diabetes and type 2 diabetes. Type 1 diabetes, or insulin-dependent Diabetes mellitus (IDDM), is defined by development of ketoacidosis in the absence of insulin therapy. Type 1 diabetes most often manifests in childhood and is therefore also called juvenile onset diabetes. Rapid in onset and progress, it accounts for about 10 to 15 percent of all cases. Type 2 diabetes, or non-insulin-dependent Diabetes mellitus (NIDDM), is characterized by persistent hyperglycaemia but rarely by ketoacidosis. Type 2 diabetes typically manifests after age 40 and progresses slowly. Due to its late onset, it has formerly been called adult-onset diabetes. Type 2 diabetes can result from genetic defects that cause both insulin resistance and insulin deficiency. Type 2 diabetes, which is by far the most frequent one, is often not accompanied by clinical illness in its initial stages and is detected instead by elevated blood or urine glucose levels.

Diabetes type 1 and 2 are both considered as a group of disorders with multiple causes, rather than a single disorder. Common to diabetes type 1 and 2 is that entry of glucose into cells is impaired. Entry of glucose into cells is typically catalyzed by insulin, a hormone secreted by Langerhans cells in the human pancreas. By facilitating entry of sugar glucose into tissue cells of the body insulin provides energy for metabolic activities. Impairment of glucose uptake may be a result either of a deficiency in the amount of insulin produced in the body or of altered target cells not enabling the cells to take up glucose. Consequently, glucose builds up in the blood and is excreted in the urine.

In type 1 diabetes, the etiological problem is almost always a severe or total reduction in insulin production. This reduction is typically the result of an autoimmune destruction of b-cells of the pancreas, responsible for producing insulin. The most common therapy for insulin dependent Diabetes mellitus (type 1 diabetes) is the provision of insulin.

In type 2 diabetes, the pancreas often produces a considerable quantity of insulin, whereas the hormone is unable to promote the utilization of glucose by tissues. In fact, a central feature of Type 2 diabetes is insulin resistance. Onset of type 2 diabetes resulting from insulin resistance is associated with other conditions, whereby two major forms of type 2 diabetes are to be distinguished. The first form is late onset of type 2 diabetes associated with obesity and the second one late onset of type 2 diabetes not associated with obesity.

Type 2 diabetes associated with obesity presently develops in an epidemic rate and is at present of major interest. E.g. in the United States the proportion of the population under 40 that can be clinically defined as obese now exceeds 25%. Many children are obese and are developing type 2 diabetes at an alarming rate. The dramatic rise in obesity in the US has led to an equally alarming increase in the percentage of the population suffering from metabolic syndrome. The metabolic syndrome is a clustering of atherosclerotic cardiovascular disease risk factors, one of which involves insulin resistance characteristic for type 2 diabetes.

Various efforts have been made to treat diabetes and in particular insulin resistant states such as diabetes type 2. One such way of curing these conditions is to provide so called "insulin mimetics", i.e. compounds capable of "mimicking" the functions of insulin such as to enable cells to take up glucose.

Several inorganic compounds have been reported to mimic the effects of insulin, in vivo as well as in isolated cells and tissues. Such mimetics include vanadium (IV)/(V) compounds (cf. Heyliger et al., "Effect of vanadate on elevated blood glucose and depressed cardiac performance of diabetic rats", Science 1985, vol. 227, pp. 1474-7); selenates (cf. McNeill et al., "Insulin like effects of sodium selenate in streptozotrocin-induced diabetic rats", Diabetes 1991, vol. 40, pp. 1675-8), lithium salts (cf. Rodriguez-Gil et al., "Lithium restores glycogen synthesis from glucose in hepatocytes from diabetic rats", Arch. Biochem. Biophys. 1993, vol. 301, pp. 411-5), tungsten (VI) and molybdenum (VI) compounds (cf. US 5,595,763 and Jinping Li et al., Biochemistry 1995, 34, 6218-6225)).

Among the above inorganic compounds, vanadium and its derivatives have been proven as potent insulin-mimetics. There is convincing evidence for the effects of vanadates and peroxovanadium complexes (vanadium in its +5 oxidation state combined with oxygen, in particular orthovanadate VO₄³⁻, cf. US 4,882,171), and vanadyl VO²⁺ salts and complexes (vanadium in its +4 oxidation state; cf. US 5,300,496) to increase the cells' susceptibility for glucose uptake. Vanadium compounds are currently undergoing clinical trials in Europe and America. However, event though promising results for the transport of glucose into cells have been gathered, administration of vanadium compounds is accompanied by serious toxicity problems at effective doses. Administered concentrations must be close to toxic levels, if desired insulin-mimetic effects in animals are to be achieved. Considerable side effects are observed for vanadium-treatment that are independent upon the chemical nature of the specific vanadium used for therapy (cf. Domingo et al., "Oral vanadium administration to streptozocin-diabetic rats has marked negative side-effects wich are independent of the form of vanadium used", Toxicology 1991, vol. 66, pp. 279-87.). Serious problems with vanadium compounds toxicity are observed at any kind of dosage suitable for lowering blood glucose levels, including a significant mortality rate.

Tungstate (VI) compounds have also been tested as insulin mimicking compounds, (see e.g. US 5,595,763). US 5,595,763 discloses tungstate (VI) compounds and compositions containing those compounds for oral treatment of diabetes mellitus, i.e. type I or type II diabetes mellitus. According to US 5,595,763 tungstate (VI) compounds were 40-times less toxic than vanadate (V) compounds, when applied orally, and 30-times less toxic, when applied systemically. However, positive effects of the tungstate (VI) compounds used in US 5,595,763 are also observed close to toxic levels. These compounds appear to severely stress the immune system and, therefore, the dosage has to be lowered.

Molybdate compounds have been shown to activate several insulin bioeffects via insulin-independent pathways (see Shisheva and Shechter, 1991, FEBS Lett. 300, 93-96). However, also molybdate facilitates bioeffects in rat adipocytes only at high (millimolar) concentrations.

Li et al. (1995, supra) disclose permolybdate (pMo, peroxide of molybdate) and pertungstate (pW, peroxide of tungstate) compounds as stimulators of insulin effects in rat adipocytes, and being active in inducing normoglycemia in STZ-rats. Li et al. (1995) suggest, that permolybdates and pertungstates mimick insulin effects via the insulin receptor in a hormone independent intracellular mode. Therein, it was demonstrated pW and pMo displayed improved results versus tungstates and molybdates in a concentration dependent stimulation of lipogenesis, but less efficient than Vanadates. Additionally, pW and pMo have to be administered in millimolar doses.

In an alternative approach, transport of glucose into cells is mediated by using vanadate in combination with substrates of semicarbazide-sensitive amine-oxidase (SSAO), such as benzylamine or tyramine. As reported by Enrique-Tarancon et al. (G. Enrique-Tarancon et al., J. Biol.. Chem. 1998, No. 14, 3, pp. 8025-8032 and G. Enrique-Tarancon et al., Biochem. J. 2000, vol. 350, pp. 171-180 or WO 02/38152) a combination of amines such as benzylamine or tyramine with vanadate is suitable to stimulate glucose transport and GLUT4 translocation in (rat) 3T3-L1 adipocytes. According to Enrique-Tarancon et al. (1998, 2000, supra) glucose transport is stimulated by an increase of GLUT4 carrier concentration on the cell surface, resulting from potent tyrosine phosphorylation. Similarly, Marti et al. (Journal of Pharmacology and Experimental Therapeutics 285:342-349, 1998) report on the stimulation of glucose transport by using a combination of tyramine and vanadate. According to Marti et al. (1998, supra) stimulation of glucose transport was sensitive to MAO (monoamine oxidase) and SSAO inhibitors and to catalase. Marti et al (1995, supra) also disclose the use of vanadate in combination with tyramine. However, it is desirable to lower the doses for vanadate as much as possible in order to avoid negative side effects of such a treatment due to toxicity of vanadate.

Due to the above, there is need in the art to provide effective insulin mimetics for the treatment and/or prevention of diabetes, particularly for diabetes type II, being effective at low doses and having minimal toxic side effects

The object of the present invention is solved by a (pharmaceutical) composition for simultaneous, separate or sequential administration, comprising a pharmaceutically acceptable compound derived from the Vla and Vlb groups of the periodic table and a pharmaceutically acceptable amine selected from the group consisting of semicarbazide-sensitive amine oxidase (SSAO) substrates, or a pharmaceutically acceptable salt of said amine, in admixture with pharmaceutically acceptable excipients or carriers.

A compound derived from the VIa and VIb groups of the periodic table as contained in the inventive (pharmaceutical) composition is preferably a chemical compound comprising, as a part of its structure, a metal ion from the VIa and VIb groups of the periodic table. This metal ion is preferably selected from the metals selenium, molybdenum and/or tungsten. A chemical compound derived from the VIa and VIb groups of the periodic table may occur in a charged or a non-charged form, whereby the chemical compound typically comprises the metal ion and further elements of the periodic table such as to form e.g. a salt of the respective metal ion. If the salt is positively charged, the salt may further comprise a counter ion, which is then negatively charged, e.g. F⁻, CL⁻, Br⁻, I⁻, OH⁻, etc., or any pharmaceutically acceptable organic or inorganic ionic species which carries a negative charge. If the salt is negatively charged, the salt may further comprise a counter ion, which is positively charged. Positively charged counter ions typically comprise metals from alkali- or earth alkali metals, such as sodium, potassium, magnesium, calcium, as well as other positively charged ions such as ammonium or any pharmaceutically acceptable organic or inorganic ionic species which carries a positive charge. If the positively or negatively charged salt is combined with a negatively or positively charged counter ion, preferably a neutral salt is obtained.

The chemical compounds derived from the Vla and Vlb groups of the periodic table are preferably derived from any of the selenium, molybdenum and/or tungsten compounds as disclosed below.

Selenium (Se) typically occurs in the inventive (pharmaceutical) composition in its oxidation state (VI+). In the inventive composition, selenium, preferably Se⁶⁺, may occur along with in any chemical entity. Typically, the cation Se⁶⁺ occurs in the inventive (pharmaceutical) composition as a selenium compound selected from selenates (VI), selenyl salts and/or selenyl complexes. Selenium compounds preferably occur in aqueous solution in the form of their selenates, such as SeO₄²⁻ or HSeO₄⁻, in the form of their diselenates Se₂O₇²⁻, HSe₂O₇⁻, or as the corresponding halogenselenates, such as XSeO₃H, or halogendiselenates. In the inventive composition selenates also may present the form of their salts, e.g. such as [M⁺][SeO₄²⁻]⁻, [M⁺]₂[SeO₄²⁻], [M²⁺] [SeO₄²⁻]- or HSeO₄⁻, in the form of the diselenates [M⁺] [Se₂O₇²⁻]⁻, [M⁺]₂[Se₂O₇²⁻], [M⁺][HSe₂O₇⁻], whereby M is preferably a metal, which may be selected from any alkaline or alkaline earth metal, preferably Na⁺, K⁺, Mg²⁺, Ca²⁺, etc.. Selenium compounds, as used in the inventive (pharmaceutical) composition, may further occur as selenylhalogenides or oxyselenylhalogenides; e.g. as SeX₆, or O₂SeX₂ whereby X = F, Cl, Br or I. Alternatively, selenium compounds as used in the inventive composition may be attached to alcohols, amines, thiols, carboxyclic acids, amines, amino acids, heterocycles containing N or O, etc.. Selenates also may occur in form of selenocysteins, preferably as selenopeptides, comprising 3 to 30, more preferably 5-20 amino acids. Preferably, these selenopeptides, containing at least one selenocystein, are capable of forming dimers , e.g. by generating disulfide bridges between the terminal selenes of selenocysteins of different selenopeptides. Thus, selenopetides may occur in their monomeric form as well as a dimer, etc. multimer.

Molybdenum (Mo) typically occurs in the inventive (pharmaceutical) composition in its oxidation state (VI+), preferably with a tetraedric or octaedric coordination sphere. In the inventive composition, molybdenum, preferably Mo⁶⁺, may occur along with in any chemical entity. The cation Mo⁶⁺ preferably occurs as a molybdenum compound selected from molybdates (VI), permolybdates, molybdyl salts and/or molybdyl complexes. The cation Mo⁶⁺ has never been observed isolated, and it is always accompanied with a chemical moiety partially formed by a coordination sphere around the atom of Mo(VI). The coordination sphere can be formed by inorganic ligands (oxide, hydroxide, peroxide, etc) as, for example, in the case of the molybdate anion (coordination sphere formed by four oxide ions), or in the case of peroxymolybdates (coordination sphere formed by mixtures of oxide and peroxide ions). The coordination sphere can also be formed by organic ligands which are molecules or ions attached to Mo(VI) atom through O, S or N atoms belonging to different pharmaceutically acceptable organic compounds (e.g. pharmaceutically acceptable alcohols, thiols, carboxylic acids, amines, aminoacids, N-containing heterocycles, etc). Mixed inorganic/organic coordination spheres are also possible. When the structure formed by the Mo(VI) atom and its coordination sphere is not neutral, the term "chemical moiety" also includes any pharmaceutically acceptable ionic species which renders the whole molybdenum (VI) compound neutral. For example, the molybdenum anion is always accompanied by a cation (e.g. ammonium, sodium, potassium, magnesium, calcium) to form a neutral molybdenum salt. In aqueous solutions, Mo⁶⁺ typically occurs in the form of hydrates of MoO₂²⁺, MoO₃·nH₂O (n=1-2), etc. Molybdate (VI) compounds may occur here as mono-molybdates, such as MoO₄²⁻, or as polymolybdates ("isomolybdates"), such as e.g. heptamolybdates Mo₇O₂₄⁶⁻, octamolybdates Mo₈O₂₆⁴⁻ or decamolybdates Mo₁₀O₃₄⁸⁻, e.g. (NH₄)₈Mo₁₀O₃₄ These mono- and/or polymolybdates also may be present in the inventive composition in the form of their respective salts, e.g. ammonium- or halogenide salts such as [MoO₂Cl₂(H₂O)₂] or [3(NH₄)₂MoO₄ 4MoO₃], etc.. Isopolymolybdates also may be formed by Mo⁶⁺, typically comprising the form MoO₃(OH)⁻ or hexahydroxymolybdates (orthomolybdenum acid) H₆MoO₆ = Mo(OH)₆. These forms may rearrange by condensation to Mo(OMoO₃)₆⁶⁻, which may rearrange to heptamolybdate (see above). Additionally, Mo⁶⁺ also may occur as an oxide MoO₃. Permolybdates (pMoOₓ) of Mo also may be formed, preferably by reacting molybdenum with H₂O₂. Finally, Mo⁶⁺ may be present in the inventive (pharmaceutical) composition as an halogenide MoX₆, as an Mo⁶⁺-halogenide oxide MoOX₄ or MoO₂X₂, whereby X = F, Cl, Br or I.

Tungsten (W) is typically present in the inventive (pharmaceutical) composition in its oxidation state (+5) or (+6), preferably with a tetraedric or octaedric coordination sphere. In the inventive composition, tungsten, preferably W⁵⁺ or W⁶⁺, may occur along with any chemical entity. The cations W⁵⁺ or W⁶⁺ preferably occur as tungsten compounds selected from tungstates (V)/(VI), pertungstates, tungstyl salts and/or tungstyl complexes. The cation W⁶⁺ has never been observed isolated, and it is always accompanied with a chemical moiety partially formed by a coordination sphere around the atom of W(VI). The coordination sphere can be formed by inorganic ligands (oxide, hydroxide, peroxide, etc) as, for example, in the case of the tungstate anion (coordination sphere formed by four oxide ions), or in the case of peroxytungstates (coordination sphere formed by mixtures of oxide and peroxide ions). The coordination sphere can also be formed by organic ligands which are molecules or ions attached to W(VI) atom through O, S or N atoms belonging to different pharmaceutically acceptable organic compounds (e.g. pharmaceutically acceptable alcohols, thiols, carboxylic acids, amines, amino acids, N-containing heterocycles, etc). Mixed inorganic/organic coordination spheres are also possible. When the structure formed by the W(VI) atom and its coordination sphere is not neutral, the term "chemical moiety" also includes any pharmaceutically acceptable ionic species which renders the entire tungsten (VI) compound neutral. For example, the tungstate anion is always accompanied by a cation (e.g. ammonium, sodium, potassium, magnesium, calcium) to form a neutral tungstate salt. In aqueous solutions, W⁶⁺ typically occurs in the form of mono-tungstates (M^{II}WO₄), whereas M" is a metal selected from earth alkali metals, e.g. Mg²⁺ or Ca²⁺; Poly-tungstates (e.g. HW₆O₂₁⁵⁻), such as hexatungstates H₇W₆O₂₄⁵⁻, decatungstates W₁₀O₃₄⁸⁻, dodecatung-states H₂W₁₂O₄₀⁶⁻ or polytungstates H₂W₁₂O₄₂¹⁰⁻, may also occur. Alternatively, tungstates may be heteropolyacids, such as [A(W₆O₂₄)]ⁿ⁻¹²; whereas A is a heteroatom (or not present) and n = valences of heteroatom A; Pertungstates (pWOₓ) of W also may be used, preferably by reacting tungsten with H₂O₂. Finally, W⁶⁺ may be present in the inventive (pharmaceutical) composition as an halogenide WX₆, as an W⁶⁺-halogenide oxide WOX₄ or WO₂X₂, whereby X = F, Cl, Br or I. Furthermore, tungstate may occur as isopolytungstates (paratungstates, metatungstates, etc) which differ in their aggregation status; their use is also contemplated in this invention. Hydrates of tungsten (VI) compounds are common (e.g. the dihydrate of sodium tungstate), and their use is also considered to be within of this invention.

Any of the afore mentioned compounds derived from the VIa and VIb groups of the periodic table may be present in the inventive (pharmaceutical) composition in a soluble and or solubilized form. Therefore, the selenium (VI), molybdenum (VI) and/or tungsten (VNI) compounds and their (cationic) moieties as contained in the inventive (pharmaceutical) composition are preferably selected such as to obtain a soluble compound.

Furthermore, compounds derived from the Vla and Vlb groups of the periodic table are preferably pharmaceutically acceptable compounds. In the context of this invention, the term "a pharmaceutically acceptable compound, derived from the Vla and Vlb groups of the periodic table", is intended to include any of the aforementioned compounds, derived from the Vla and Vlb groups of the periodic table, attached to a chemical structure that is pharmaceutically acceptable by itself. The term "pharmaceutically acceptable compound derived from the VIa and VIb groups of the periodic table" also includes any pharmaceutically acceptable solvate (e.g. hydrate) of the compounds as contained in the inventive compositions.

In additional to compounds, derived from the VIa and VIb groups of the periodic table, the inventive (pharmaceutical) composition further contains a preferably pharmaceutically acceptable SSAO substrate as another component. Such SSAO substrates may be selected from any of the substrates of a semicarbazide-sensitive amine oxidase (SSAO). In the meaning of the present invention a "semicarbazide-sensitive amine oxidase" (SSAO) represents any enzyme present in tissue of mammals which is able to oxidize a broad variety of amines with the concomitant production of hydrogen peroxide, and which is inhibited by semicarbazide. Apart from some few secondary amines, most substrates of semicarbazide-sensitive amine oxidases are primary amines, such as tyramine, benzylamine, 2-(4-fluoro-phenyl)-ethylamine, 4-fluoro-benzylamine, 3-phenyl-propylamine, 4-phenyl-butylamine, 2,3-dimethoxybenzylamine, 1-naphtalenemethylamine, deoxyepinephrine, epinephrine, norepinephrine, dopamine, histamine, β-phenylethylamine, N-acetylputrescine, tryptamine, n-octylamine, n-pentylamine, kynuramine, 3-methoxytyramine, and n-decylamine.

A, pharmaceutically acceptable SSAO substrate may also be derived from general formula (I): 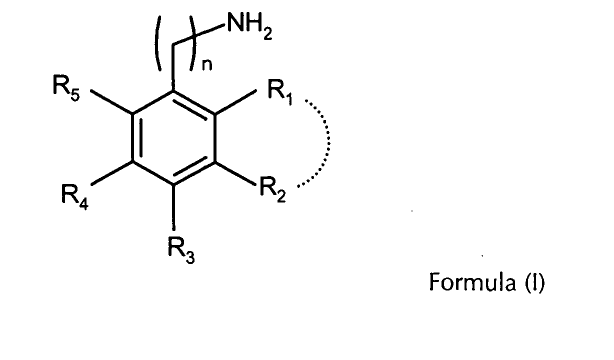 wherein:
- R¹, R², R³, R⁴ and R⁵: independent from each other represent a bond or are selected from the group consisting of:
H, O, N,
OH, OR⁶,
X, CX₃, CHX₂, CH₂X, CR⁶X₂, CR₂⁶X, CR₃⁶, OCX₃,
CN, CO, COOH, COOR⁶,
NH, NH₂, NHR⁶, NR⁶R⁷, NO, NO₂, NOH, NOR⁶,
(CH₂)ₚNR⁸R⁹, O(CH₂)_{q}Ph, CONR¹⁰R¹¹, COR¹²,
CH₂NHC(=NH)NH₂;
CH₃, (CH₂)ₙ, (CH₂)ₙCH₃, (CH₂)ₙ R⁶, OCH₃, O(CH₂)ₙ,
O(CH₂)ₙCH₃; O(CH₂)ₙ R¹, (CH₂)ₙOH, or
a branched or linear C₁-C₆-alkyl, C₁-C₆-alkenyl, C₁-C₆₋heteroalkyl, C₁-C₆-heteroalkenyl, C₁-C₆-alkoxy, C₁-C₆-alkenoxy, C₁-C₆-acyl, C₁-C₁₄-cycloalkyl, C₁-C₁₄-cycloalkenyl, C₁-C₁₄-aryl, C₁-C₁₄-heteroaryl, arylalkyl, arylalkenyl, C₅₋₁₄-aryloxy, heteroarylalkyl, heteroarylalkenyl, heterocycloalkyl, heterocycloalkenyl, carboxamido-, acylamino-, amidino-, or heteroaryloxy moiety; and
   wherein
   X = F, Cl, Br, I, or CN or CO
   p and q are integers from 1 to 3;
   n is an integer from 1 to 4; and
   R⁶, R⁷, R⁸, R⁹, R¹⁰, R¹¹ and R¹² are independently selected from the group consisting of: H, O, N, OH, X, CX₃, CHX₂, CH₂X, CR⁶X₂, CR₂⁶X, CR₃⁶, CN, CO, COOH, NH, NH₂, NO, NO₂, NOH, or CH₃, (CH₂)ₙ, (CH₂)ₙCH₃, OCH₃, O(CH₂)ₙ, O(CH₂)ₙCH₃, (CH₂)ₙOH, and/or a branched or linear (C₁-C₆)-alkyl, C₁-C₆₋alkyl, C₁-C₆-alkenyl, C₁-C₆-heteroalkyl, C₁-C₆heteroalkenyl, C₁-
C₆-alkoxy, C₁-C₆-alkenoxy, C₁-C₁₄-acyl, C₁-C₁₄-cycloalkyl, C₁₋C₁₄-cycloalkenyl, phenyl, C₁-C₁₄-aryl, C₁-C₁₄-heteroaryl, arylalkyl, arylalkenyl, C₅₋₁₄-aryloxy, heteroarylalkyl, heteroarylalkenyl, heterocycloalkyl, heterocycloalkenyl, carboxamido-, acylamino-, amidino-, or heteroaryloxy moiety; and/or
- R¹ and R²: are bound together forming a ring, wherein R¹ and R² represent a bond or are selected from the group as defined above:

Preferably, R₁, R₂, R₃, R₄ and R₅ of formula (I) are independent from each other H, (C₁-C₆)-alkyl, OH, (C₁-C₆)-alkoxy, O(CH₂)_{q}Ph, CF₃, OCF₃, F, Cl, Br, and NO₂, wherein R₁ and R₂ may be bound together forming a ring, wherein R¹ and R² represent a bond or are selected from the group as defined above.

More preferably compounds of formula (I) are defined as above, wherein n = 1.

While any amine being an SSAO substrate may be used as a component of the inventive composition, primary amines are particularly preferred.

In a further embodiment the present invention pharmaceutical compositions for the treatment of diabetes, particularly for diabetes mellitus, more preferably for insulin resistant states, e.g. diabetes type 2 or diseases related thereto, are provided. Preferably, the (pharmaceutical) composition according to the invention is an antidiabetic medicament. Pharmaceutical compositions according to the present invention typically comprise the inventive combination of SSAO substrates and pharmaceutically acceptable compounds of the Vla and Vlb groups of the periodic table, i.e. selenium (Vl)-, molybdenum (Vl)- and tungsten (V)/(VI) compounds, etc.. The inventive pharmaceutical compositions are suitable for the treatment of diabetes in prior, simultaneous or subsequent administration. Typically, these pharmaceutical compositions are administered orally or parenterally, including the intravenous, intramuscular, intraperitoneal, subcutaneous, rectal and topical routes of administration.

Toxicity and therapeutic efficacy of the inventive (pharmaceutical) composition comprising SSAO substrates and pharmaceutically acceptable compounds of the VIa and VIb groups of the periodic table, i.e. selenium (VI)-, molybdenum (VI)- and tungsten (V)/(VI) compounds, etc. may be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index, which may be expressed as the ratio LD₅₀/ED₅₀. Inventive compounds (or compositions) which exhibit large therapeutic indices are preferred. While compounds that potentially exhibit toxic side effects may be used, care should be taken to design a delivery system that targets such reagents to the site of affected tissue in order to minimize potential damage to normal cells and, thereby, reduce side effects.

Data obtained from cell culture assays and animal studies can be used in formulating a range of dosage for use in humans. The dosage of the inventive compounds to be administered lies preferably within a range of circulating concentrations that include the ED₅₀ with little or no toxicity. The dosage may vary within this range depending upon the dosage form employed and the route of administration utilized. For any inventive compounds to be administered in the present invention, the therapeutically effective dose can be estimated initially from cell culture assays. A dose may be formulated in animal models to achieve a circulating plasma concentration range that includes the IC₅₀ (i.e., the concentration of the test compound which achieves a half- maximal inhibition of activity) as determined in cell culture. Such information can be used to more accurately determine useful doses in humans. The compounds of the invention preferably have an IC₅₀ less than 10 µM, which can be determined by biochemical or cellular assays typically used in the art. Some compounds of the invention are effective at concentrations of 1-10 mM. Based on these numbers, it is possible to derive an appropriate dosage for administration to subjects. Formation of prodrugs is well known in the art in order to enhance the properties of the parent compound. Such properties include solubility, absorption, biostability and release time (see "Pharmaceutical Dosage Form and Drug Delivery Systems" (Sixth Edition), edited by Ansel et al., publ. by Williams & Wilkins, pgs. 27-29, (1995)). Commonly used prodrugs of the inventive compounds can be designed to take advantage of the major drug biotransformation reactions and are also to be considered within the scope of the invention. Major drug biotransformation reactions include N-dealkylation, O-dealkylation, aliphatic hydroxylation, aromatic hydroxylation, N-oxidation, S-oxidation, deamination, hydrolysis reactions, glucuronidation, sulfation and acetylation (see Goodman and Gilman's The Pharmacological Basis of Therapeutics (Ninth Edition), editor Molinoff et al., publ. by McGraw-Hill, pages 11-13, (1996)). The pharmaceutical compositions according to the present invention can be prepared such that they may be administered orally, parenterally, nasally, sublingually, rectally or vaginally. Parenteral administration includes intravenous, intraarticular, intramuscular, intraperitoneal, and subcutaneous injections, as well as use of infusion techniques. One or more compounds of the invention may be present in association with one or more non-toxic pharmaceutically acceptable ingredients and optionally, other active anti-proliferative agents, to form the inventive pharmaceutical composition. These compositions can be prepared by applying known techniques in the art such as those taught in Remington's Pharmaceutical Sciences (Fourteenth Edition), Managing Editor, John E. Hoover, Mack Publishing Co., (1970) or Pharmaceutical Dosage Form and Dug Delivery Systems (Sixth Edition), edited by Ansel et al., publ. by Williams & Wilkins, (1995).

As indicated above, pharmaceutical compositions containing the inventive combination of SSAO substrates and pharmaceutically acceptable compounds of the VIa and VIb groups of the periodic table, i.e. selenium (VI)-, molybdenum (VI)- and tungsten (V)/(VI) compounds, etc. may be in a form suitable for oral use, for example, as tablets, troches, lozenges, aqueous or oily suspensions, dispersible powders or granules, emulsions, hard or soft capsules, or syrups or elixirs. Inventive pharmaceutical compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically acceptable preparations. Tablets typically contain the inventive combination of SSAO substrates and pharmaceutically acceptable compounds of the VIa and VIb groups of the periodic table, i.e. selenium (VI)-, molybdenum (VI)- and tungsten (V)/(VI) compounds, etc. in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia; and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropylmethyl-cellulose or hydroxypropylcellulose, or a time delay material such as ethyl cellulose, cellulose acetate buryrate may be employed.

Formulations for oral use according to the present invention may also be presented as hard gelatin capsules wherein the inventive (pharmaceutical) composition comprising SSAO substrates and pharmaceutically acceptable compounds of the VIa and VIb groups of the periodic table, i.e. selenium (VI)-, molybdenum (VI)- and tungsten (V)/(VI) compounds, etc. is typically mixed with an inert solid diluent, for example, calcium carbonate, calcium phosphate or kaolin, or as soft gelatin capsules wherein the inventive (pharmaceutical) composition mixed with water soluble carrier such as polyethyleneglycol or an oil medium, for example peanut oil, liquid paraffin, or olive oil.

Aqueous suspensions contain the inventive combination of SSAO substrates and pharmaceutically acceptable compounds of the Vla and Vlb groups of the periodic table, i.e. selenium (VI)-, molybdenum (Vl)- and tungsten (V)/(VI) compounds, etc. in admixture with excipients suitable for the manufacture of aqueous suspensions. Such excipients are suspending agents, for example sodium carboxymethylcellulose, methylcellulose, hydroxypropylmethyl- cellulose, sodium alginate, polyvinyl-pyrrolidone, gum tragacanth and gum acacia; dispersing or wetting agents may be a naturally occurring phosphatide, for example lecithin; or condensation products of an alkylene oxide with fatty acids, for example polyoxyethylene stearate; or condensation products of ethylene oxide with long chain aliphatic alcohols, for example heptadecaethylene-oxycetanol; or condensation products of ethylene oxide with partial esters derived from fatty acids and a hexitol such as polyoxyethylene sorbitol monooleate; or condensation products of ethylene oxide with partial esters derived from fatty acids and hexitol anhydrides, for example polyethylene sorbitan monooleate. The aqueous suspensions may also contain one or more preservatives, for example ethyl or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These inventive pharmaceutical compositions may be preserved by addition of an anti-oxidant such as butylated hydroxyanisol or alpha-tocopherol.

Dispersible powders and granules suitable for preparation of an aqueous suspension by the addition of water provide the inventive combination of SSAO substrates and pharmaceutically acceptable compounds of the Vla and Vlb groups of the periodic table, i.e. selenium (Vl)-, molybdenum (Vl)- and tungsten (V)/(Vl) compounds, etc. in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

Pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavouring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

Pharmaceutical compositions according to the present invention may be in the form of a sterile injectable aqueous solutions. Among the acceptable vehicles and solvents that may be employed are water, Ringer's solution and isotonic sodium chloride solution.

Sterile injectable preparations may also be a sterile injectable oil-in-water microemulsion where the compound of the invention is dissolved in the oily phase. For example, the active ingredient may be first dissolved in a mixture of soybean oil and lecithin. The oil solution is then introduced into a water and glycerol mixture and processed to form a microemulation.

The injectable solutions or microemulsions may be introduced into a patient's blood stream by local bolus injection. Alternatively, it may be advantageous to administer the solution or microemulsion in such a way as to maintain a constant circulating concentration of the active compound. In order to maintain such a constant concentration, a continuous intravenous delivery device may be utilized. An example of such a device is the Deltec CADD-PLUS°° model 5400 intravenous pump.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a nontoxic parenterally acceptable diluent or solvent, for example as a solution in 1,3-butane diol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Pharmaceutical compositions according to the present invention and containing an inventive combination of SSAO substrates and pharmaceutically acceptable compounds of the Vla and Vlb groups of the periodic table, i.e. selenium (VI)-, molybdenum (Vl)- and tungsten (V)/(VI) compounds, etc., may also be administered in the form of a suppository for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatine, hydrogenated vegetable oils, mixtures of polyethylene glycols of various molecular weights and fatty acid esters of polyethylene glycol.

The pharmaceutical compositions for the present invention can be administered via transdermal routes, using those forms of transdermal skin patches well known to those of ordinary skill in the art. To be administered in the form of a transdermal delivery system, the dosage administration will preferably be continuous rather than intermittent throughout the dosage regimen.

The compounds of the invention may also be co-administered with other well known therapeutic agents that are selected for their particular usefulness against the condition that is being treated. The compounds may be administered simultaneously or sequentially.

When the pharmaceutical composition according to this invention is administered to a subject, preferably a human subject, the daily dosage will normally be determined by the prescribing physician with the dosage generally varying according to the age, weight, and response of the individual patient, as well as the severity of the patient's symptoms.

In a further embodiment, the inventive combination of SSAO substrates and pharmaceutically acceptable compounds of the VIa and VIb groups of the periodic table as defined above, i.e. selenium (VI)-, molybdenum (VI)- and tungsten (V)/(VI) compounds, etc., materials and/or reagents required for administering the compounds of the invention may be assembled together in a kit. When the components of the kit are provided in one or more liquid solutions, the liquid solution preferably is an aqueous solution, with a sterile aqueous solution being particularly preferred.

The kit may be suitable for the treatment of diabetes in a prior, simultaneous or subsequent administration. It typically comprises the inventive compositions in a single pharmaceutically acceptable composition in one ore more containers. The container means may itself be geared for administration, such as an inhalant, syringe, pipette, eye dropper, or other such like apparatus, from which the formulation may be applied to an infected area of the body, such as the lungs, or injected into an animal, or even applied to and mixed with the other components of the kit. The compositions of these kits also may be provided in dried or lyophilized forms. When reagents or components are provided as a dried form, reconstitution generally is by the addition of a suitable solvent. It is envisioned that the solvent also may be provided in another container means. The kits of the invention may also include an instruction sheet defining administration of the agent. The kits of the present invention also will typically include a means for containing the vials in close confinement for commercial sale such as, e.g., injection or blow-molded plastic containers into which the desired vials are retained. Irrespective of the number or type of containers, the kits of the invention also may comprise, or be packaged with a separate instrument for assisting with the injection/administration or placement of the ultimate complex composition within the body of an animal. Such an instrument may be an inhalant, syringe, pipette, forceps, measured spoon, eye dropper or any such medically approved delivery vehicle. Other instrumentation includes devices that permit the reading or monitoring of reactions or amounts of compounds or polypeptides.

The inventive (pharmaceutical) composition comprising SSAO substrates and pharmaceutically acceptable compounds of the Vla and Vlb groups of the periodic table, i.e. selenium (Vl)-, molybdenum (Vl)- and tungsten (V)/(Vl) compounds, etc., as well as the inventive pharmaceutical compositions may be used for the treatment of diabetes, particularly for the treatment of insulin resistant forms of diabetes, such as dibetes type 2. Hereby, the inventive (pharmaceutical) composition comprising SSAO substrates and pharmaceutically acceptable compounds of the VIa and VIb groups of the periodic table, i.e. selenium (VI)-, molybdenum (VI)- and tungsten (V)/(VI) compounds, etc., as well as the inventive pharmaceutical compositions is administered by a method disclosed above to the patient (animal or human). Dosage requirements are dependent e.g. upon the age, the body weight, gender, the method of administration (e.g. orally or parenterally) and the severity of the disease to be treated.

For the purpose of the present invention it is crucial that the effective concentrations of the inventive (pharmaceutical) composition comprising SSAO substrates and pharmaceutically acceptable compounds of the Vla and Vlb groups of the periodic table, i.e. selenium (Vl)-, molybdenum (Vl)- and tungsten (V)/(Vl) compounds, etc. and to their use as insulin mimetics, i.e., are one order of magnitude lower (or less) than the concentrations needed to mimic insulin action when the compounds of the Vla and Vlb groups of the periodic table, i.e. selenium (VI)-, molybdenum (VI)- and tungsten (V)/(VI) compounds, etc. are used alone. This means that there is synergism between the compounds of the Vla and Vlb groups of the periodic table and the SSAO substrate as mentioned above. From a practical point of view, such synergism means that, for a given insulin mimetic effect, the toxicity of the drug based on a combination of compounds of the Vla and Vlb groups of the periodic table and SSAO substrates as mentioned above is much lower than the toxicity of a drug based on compounds of the VIa and VIb groups of the periodic table alone. This represents an important advantage of the (pharmaceutical) and pharmaceutical compositions of the present invention in respect of the compositions known in the art, for the treatment and/or prevention of Diabetes mellitus.

The term "contains" and its variations, as well as the term "containing" as used in the specification and the claims does not exclude other additives, components, elements or steps. Furthermore, the disclosure in the summary, which is attached to this application, is incorporated by reference as well in its entirety.

The following Examples are intended to illustrate the present invention without limiting its scope to these examples.

### EXAMPLES:

### Example 1:

### Preparation of 2-hydroxy-3-methoxybenzylamine

2-hydroxy-3-methoxybenzaldehyde (1.52 g, 10 mmol) was dissolved in methanol (10 ml). A solution of hydroxylamine in water at 50% (2.4 ml, 40 mmol) was added and the mixture was refluxed. After 30 min, the solvent was distilled under reduced pressure and the resulting solid was dissolved in ether, washed firstly with two volumes of saturated HCl 1N, then with saturated NaHCO₃ solution, and finally with saturated NaCl solution. The organic phase was dried with MgSO₄, and concentrated at reduced pressure to give 2-hydroxy-3-methoxybenzoxime. 2-hydroxy-3-methoxybenzoxime (83.5 mg, 0.5 mmol) and PtO₂·1H₂O (5.67 mg, 0.022 mmol) were dissolved in acetic acid (10 ml). The mixture was pressurized with H₂ to 4 bar and reacted overnight. The mixture was filtered through celite and the acetic acid was distilled under reduced pressure. The crude was washed with tetrahydrofurane (R: 85%). ESI-MS: M_{calc} (C₈H₁₁O₂N): 153.8, [M+1]⁺: 154.8 m/z, [M-NH₃]⁺: 136.7 m/z. ¹H-RMN (400 MHZ, CDCl₃): 3.7 (CH₃), 4.0 (CH₂), 7.0 (m, ³H) ppm. ¹³C-RMN (400 MHz, CDCl₃): 38, 54, 111, 118, 120 ppm. HPLC: t_{R}: 3.62 min, H₂O (TFA 1 %) /ACN (TFA 1 %) 0-100% in 15 min, column C₁₈ Symmetry 300 5 µm (3.9x150), λ = 220/254 nm.

### Example 2:

### Preparation of 1-aminomethyl-n-naphthalene-2-ol

2-hydroxynaphthalene-1-carbaldehyde, (1.52 g, 10 mmol) was dissolved in methanol (10 ml). A solution of hydroxylamine in water at 50% (2.4 ml, 40 mmol) is added and the mixture was refluxed. After 30 min, the solvent was distilled under reduced pressure and the resulting solid was dissolved in ether, washed firstly with two volumes of saturated HCl 1N, then with saturated NaHCO₃ solution, and finally with saturated NaCl solution. The organic phase was dried with MgSO₄, and concentrated at reduced pressure to give the oxime of 2-hydroxy-naphthalene-1-carbaldehyde.

The oxime of 2-hydroxy-naphthalene-1-carbaldehyde (94 mg, 0.5 mmol) and PtO₂·1H₂O (5.67 mg, 0.022 mmol) was dissolved in acetic acid (10 ml). The mixture was pressurized with H₂ to 4 bar and reacted during all night. It was filtered through celite and the acetic acid was distilled under reduced pressure. The crude mixture was washed with tetrahydrofurane. (R: 94%). ESI-MS: M_{calc} (C₈H₁₁O₂N): 173.8, [M+1]⁺: 174.8 m/z, [M-NH₃]⁺: 157.7. ¹H-RMN (400 MHz, CDCl₃): 4.3 (CH₂), 7.2 (CH), 7.3 (CH), 7.7 (CH), 8.0 (CH) ppm. ¹³C-RMN (400 MHz, CDCl₃): 38, 111, 122, 124, 128, 132, 134, 138, 160 ppm. HPLC: t_{R}: 4.4 min, H₂O(TFA 1%) / ACN (TFA 1 %) 0-100% in 15 min, column C18 Symmetry 300 5 µm ( 3.9x150), λ = 220/254 nm.

### Example 3:

### Preparation of 2-hydroxy-3-methoxybenzylamine

The title compound was obtained by solid-phase synthesis. Solid-phase manipulations were performed in polypropylene syringes fitted with a polyethylene porous disc. Solvents and soluble reagents were removed by filtration. 2-hydroxy-3-methoxybenzaldehyde (15 equiv, 250 mg) was condensed on a Rink resin (100 mg, 1.1 mmol/g) using TMOF (1 ml) as a solvent and the mixture was stirred overnight under Ar at 25 °C to give the aldimine. The resin was filtered off and washed with TMOF (5x1 min) and dry THF (5x1 min). Then, reduction of the imine was carried out with LiBH₄ (15 equiv, 40 µl) in dry THF under Ar and the mixture was stirred for 5 h at 65 °C to give the corresponding secondary amine. The resin was filtered off and washed several times with THF, H₂O, MeOH and DCM. The amine was cleaved from the resin with 5% TFA in DCM for 5 h at 25 °C. The solution was filtered and evaporated under N₂ to dryness and the compound of the title was obtained with 98 % of purity. MS: [(M+H)⁺_{calc.} 153, (M+H)⁺ₑₓₚ 154.0 m/z] and [(M-NH₃)⁺ₑₓₚ 137.0 m/z]. HPLC: t_{R}: 3.9 min., H₂O (0.1% TFA) / ACN (0.1% TFA) 0-100% in 15 min, column C₁₈ Symmetry 300 5 µm (3.9x150), λ = 220/254 nm.

### Example 4:

### Preparation of 2-hydroxynaphthylmethylamine

The title compound was obtained following a procedure analogous to the one described in Example 3 from 2-hydroxy-1-naphthaldehyde (15 equiv, 283 mg). Purity: 93%. ESI-MS: [(M+H)⁺_{calc} 173, (M+H)⁺ₑₓₚ 174 *mlz* ] and [(M-NH₃)⁺ₑₓₚ 156.9 *mlz*]*.* HPLC: t_{R}: 5.3 min., H₂O (0.1 % TFA) /ACN (0.1 % TFA) 0-100% in 15 min, column C₁₈ Symmetry 300 5 µm (3.9x150), λ = 220/254 nm.

### Example 5:

### Preparation of 2,3-dimethoxybenzaldehyde (intermediate for the preparation of 2,3-dimethoxybenzylamine)

2-hydroxy-3-methoxybenzaldehyde (3.28 mmol, 500 mg) was dissolved in 20 I DMF and then K₂CO₃ (3.28 mmol, 453 mg) was added. The mixture was stirred at room temperature for 15 min. Then, Mel (3.28 mmol, 193 µl) was added and the mixture was stirred overnight at room temperature. Afterwards, it was concentrated under reduced pressure and then diluted with 50 ml of 5% NaOH aqueous solution. The modified aldehyde was extracted with DCM (3x50 ml), dried with MgSO₄ and concentrated under reduced pressure. ESI-MS: [(M+H)⁺_{calc}:166.1, (M+H)⁺ₑₓₚ: 167.8 m/z]. ¹H-RMN (400 MHz,CDCl₃): 3.73 (2(OCH₃)), 7.0 ( m, ³H) ppm. ¹³C-RMN (400 MHz, CDCl₃): 56, 112, 118, 120, 124, 130, 154 ppm. HPLC: t_{R}: 8.1 min, gradient H₂O (0.1% TFA) / ACN (0.1% TFA) 0-100% in 15 min, column C₁₈ Symmetry 300 5 µm (3.9x150), λ = 220/254 nm.

### Example 6:

### Preparation of 2,3-dimethoxybenzylamine

Solid-phase manipulations were performed in polypropylene syringes fitted with a polyethylene porous disc. Solvents and soluble reagents were removed by filtration. 2,3-dimethoxybenzaldehyde (15 equiv) was condensed on a H₂N-XAL-MBHA resin (38 mg, 0.7 mmol/g) using TMOF (1 ml) as a solvent and the mixture was stirred overnight under Ar at 25 °C to give the aldimine. Then, the resin was filtered off and washed with TMOF (5x1 min.) and dry THF( 5x1 min.). The reduction was carried out with LiBH₄ (15 equiv, 40 µl) in dry THF under Ar and the mixture was stirred for 5 h at 65 °C to give the corresponding secondary amine. The resin was filtered off and washed several times with THF, H₂O, MeOH and DCM. The amine was cleaved from the resin with 5% TFA in DCM for 5h at 25 °C. The solution was filtered and evaporated under N₂ to dryness. ESI-MS: [(M+H)⁺_{calc} 167.2, (M+H)⁺ₑₓₚ 167.7], and [(M-NH₃)⁺ₑₓₚ 150.7]. HPLC: t_{R}: 4.3min, H₂O (0.1% TFA)/ ACN (0.1% TFA) 0-100% in 15 min, column C₁₈ X-Terra 15 µm (4.6x10), λ = 220 / 254 nm.

### Example 7:

### Preparation of 2-methoxynaphthylaldehyde (Intermediate for Example 8)

The compound of the title was obtained following a procedure analogous to the one described in Example 5 from 2-hydroxynaphthaldehyde. 2-hydroxynaphthaldehyde (2.90 mmol, 500 mg) was dissolved in 20 ml DMF and K₂CO₃ (2.90 mmol, 316 mg) was added. The mixture was stirred at room temperature for 15 min. Then, Mel (2.90 mmol, 147 µl) was added and the mixture was stirred overnight at room temperature. It was concentrated under reduced pressure and diluted with 50 ml of 5 % NaOH aqueous solution. The modified aldehyde was extracted with DCM (3x50 ml), dried with MgSO₄ and concentrated under reduced pressure. ESI-MS: [(M+H)⁺_{calc}: 186.1, (M+H)⁺ₑₓₚ: 187.8 m/z]. ¹H-RMN (400 MHz, CDCl₃): 3.73 (OCH₃), 7.2 (CH), 7.3 (CH), 7.7 (CH), 8.0(CH) ppm. ¹³C-RMN (400 MHz, CDCl₃): 56, 111, 122, 124, 128, 132, 134,138, 160 ppm. HPLC: t_{R}: 10.2 min, gradient H₂O (0.1% TFA) / ACN (0.1% TFA) 0-100% in 15 min, column C₁₈ Symmetry 300 5 µm (3.9x150), λ = 220 / 254 nm.

### Example 8:

### Preparation of 2-methoxynaphthylamine

Solid-phase manipulations were performed in polypropylene syringes fitted with a polyethylene porous disc. Solvents and soluble reagents were removed by filtration. 2-methoxynaphthaldehyde (15 equiv) was condensed on a H₂N-XAL-MBHA resin (29 mg, 0.7 mmol/g) using TMOF (1 ml) as a solvent and the mixture was stirred overnight under Ar at 25 °C to give the aldimine. The resin was filtered off and washed with TMOF ( 5x1 min.) and dry THF (5x1 min.). Then the reduction was carried out with LiBH₄ (15 equiv, 40 µl) in dry THF under Ar and the mixture was stirred for 5 h at 65 °C to give the corresponding secondary amine. The resin was filtered off and washed several times with THF, H₂O, MeOH and DCM. The amine was cleaved from the resin with 5% TFA in DCM for 5 h at 25 °C. The solution was filtered and evaporated under N₂ to dryness. ESI-MS: [(M+H)⁺_{calc} 187.2, (M+H)⁺ exp: 187.7, (M-NH₃)⁺ₑₓₚ: 170.3]. HPLC: t_{R}: 5.4 min, H₂O (0.1% TFA) / ACN (0.1% TFA) 0-100% in 15 min, column C₁₈ X-Terra 15 µm (4.6x10), λ = 220 / 254 nm.

### Example 9:

### Effects of benzylamine and selenate/molybate/tungstate compounds on glucose transport in isolated adipocytes

In order to determine whether salts formed by benzylamine and selenate/molybate/tungstate compounds produce an insulin mimetic effect, the effect of increasing concentrations of selenate/molybate/tungstate compounds on glucose transport activity in isolated rat adipocytes was tested. Stimulation of glucose transport by selenate/molybate/tungstate compounds was determined. In similar assays, activity of benzylamine and selenate/molybate/tungstate compounds on glucose transport activity in isolated rat adipocytes was analyzed. Additionally, influence activity of benzylamine and selenate/molybate/tungstate compounds on glucose transport activity in isolated rat adipocytes was analyzed in presence of semicarbazide inhibitors.

### Example 10:

### Effect of chronic administration of benzylamine and selenate/molybate/tungstate compounds in diabetic rats

Effect of chronic administration of benzylamine and selenate/molybate/tungstate compounds on glycemia from diabetic rats was determined. Thus, diabetes was induced in rats by intravenous administration of streptozotocin, which destroys the β-pancreatic cell producer of insulin. Rats were treated with buffered solution used as solvent or with benzylamine and selenate/molybate/tungstate compounds. Rats were treated four days and hyperglycemia was observed after four days of treatment, after eleven days of treatment and after fourteen days of treatment. Adipocytes of chronic treatment rats were isolated and glucose transport velocity was determined. Moreover, an inverse correlation between animal glycemia and basal glucose transport velocity was investigated in order to determine an influence of benzylamine and selenate/molybate/tungstate compounds on antidiabetic effects in adipocytes.

### Example 11:

### Effect of oral and chronic administration of benzylamine and selenate/molybate/tungstate compounds in diabetic rats

The influence of oral administration of benzylamine and selenate/molybate/tungstate compounds on glycemia from diabetic rats was studied. Thus, diabetes was induced in rats by intravenous administration of streptozotocin, and subsequently, benzylamine and selenate/molybate/tungstate compounds were administered to the rats in a unique dose. It was determined as to whether rats with treated benzylamine and selenate/molybate/tungstate compounds did or did not modify substantially its glycemia during seventeen days of treatment. Further, it was determined as to whether administration of benzylamine and selenate/molybate/tungstate compounds during seven days produced a decrease of hyperglycemia. At seven days the dose was increased to a maximum which was maintained during ten days. The influence of these doses on additional decrease in glycemia of the animals was also determined.

## Claims

1. A composition for simultaneous, separate or sequential administration, comprising a pharmaceutically acceptable compound derived from the VIa and VIb groups of the periodic table and a pharmaceutically acceptable amine selected from the group consisting of semicarbazide-sensitive amine oxidase (SSAO) substrates, or a pharmaceutically acceptable salt of said amine, optionally in admixture with pharmaceutically acceptable excipients or carriers.

2. The composition according to claim 1, wherein the compound derived from the VIa and VIb groups of the periodic table is selected from the metals selenium (VI), molybdenum (V)/(VI) and/or tungsten (V).

3. The composition according to claim 2, wherein the compounds derived from the VIa and VIb groups of the periodic table are selected from selenates (VI), selenyl salts and selenyl complexes, molybdates (VI), peroxomolybdenum complexes, molybyl salts and molybdyl complexes, tungstates (V/VI), peroxotungsten complexes, tungstyl salts and/or tungstyl complexes.

4. The composition according to claim 3, wherein the compounds derived from the Vla and Vlb groups of the periodic table are selected from the group of selenates (VI), molybdates (VI) and/or tungstates (VI).

5. The composition according to any of the claims 1-4, wherein the amine is selected from the group consisting of tyramine, benzylamine, 2-(4-fluorophenyl)-ethylamine, 4-fluoro-benzylamine, 3-phenyl-propylamine, 4-phenyl-butylamine, 2,3-dimethoxybenzylamine, 1-naphtalenemethylamine deoxyepinephrine, epinephrine, norepinephrine, dopamine, histamine, β-phenylethylamine, N-acetylputrescine, tryptamine, n-octylamine, n-pentylamine, kynuramine, 3-methoxytyramine, and n-decylamine or a compound with the following formula: 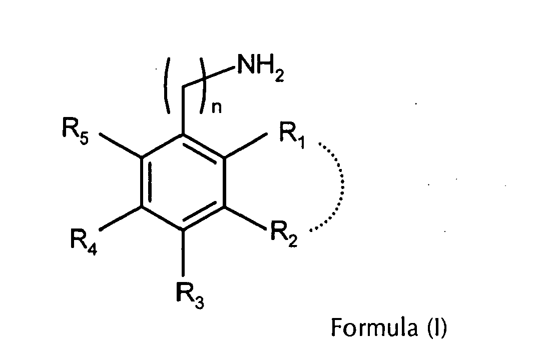 wherein:
R¹, R², R³, R⁴ and R⁵ independent from each other represent a bond or are selected from the group consisting of:
H, O, N,
OH, OR⁶,
X, CX₃, CHX₂, CH₂X, CR⁶X₂, CR₂⁶X, CR₃⁶, OCX₃,
CN, CO, COOH, COOR⁶,
NH, NH₂, NHR⁶, NR⁶R⁷, NO, NO₂, NOH, NOR⁶,
(CH₂)ₚNR⁸R⁹, O(CH₂)_{q}Ph, CONR¹⁰R¹¹, COR¹²,
CH₂NHC(=NH)NH₂;
CH₃, (CH₂)ₙ, (CH₂)ₙCH₃, (CH₂)ₙ R⁶, OCH₃, O(CH₂)ₙ,
O(CH₂)ₙCH₃; O(CH₂)ₙ R¹, (CH₂)ₙOH, or
a branched or linear C₁-C₆-alkyl, C₁-C₆-alkenyl, C₁-C₆₋heteroalkyl, C₁-C₆-heteroalkenyl, C₁-C₆-alkoxy, C₁-C₆₋alkenoxy, C₁-C₆-acyl, C₁-C₁₄-cycloalkyl, C₁-C₁₄₋cycloalkenyl, C₁-C₁₄-aryl, C₁-C₁₄-heteroaryl, arylalkyl, arylalkenyl, C₅₋₁₄-aryloxy, heteroarylalkyl, heteroarylalkenyl, heterocycloalkyl, heterocycloalkenyl, carboxamido-, acylamino-, amidino-, or heteroaryloxy moiety; and
wherein
X = F, Cl, Br, I, or CN or CO;
p and q are integers from 1 to 3;
n is an integer from 1 to 4; and
R⁶, R⁷, R⁸, R⁹, R¹⁰, R¹¹ and R¹² are independently selected from the group consisting of: H, O, N, OH, X, CX₃, CHX₂, CH₂X, CR⁶X₂, CR₂⁶X, CR₃⁶, CN, CO, COOH, NH, NH₂, NO, NO₂, NOH, or CH₃, (CH₂)ₙ, (CH₂)ₙCH₃, OCH₃, O(CH₂)ₙ, O(CH₂)ₙCH₃, (CH₂)ₙOH, and/or a branched or linear (C₁-C₆)-alkyl, C₁-C₆-alkyl, C₁-C₆₋alkenyl, C₁-C₆-heteroalkyl, C₁-C₆-heteroalkenyl, C₁-C₆₋alkoxy, C₁-C₆-alkenoxy, C₁-C₁₄-acyl, C₁-C₁₄-cycloalkyl, C₁-C₁₄-cycloalkenyl, phenyl, C₁-C₁₄-aryl, C₁-C₁₄₋heteroaryl, arylalkyl, arylalkenyl, C₅₋₁₄-aryloxy, heteroarylalkyl, heteroarylalkenyl, heterocycloalkyl, hetero-cycloalkenyl, carboxamido-, acylamino-, amidino-, or heteroaryloxy moiety; and/or
R¹ and R² are bound together forming a ring, wherein R¹ and R² represent a bond or are selected from the group as defined above.

6. The composition according to claim 5, wherein the amine is tyramine or benzylamine.

7. Composition according to any of claims 1 to 6 as an antidiabetic medicament.

8. Composition according to any of claims 1 to 6 as an anti-obesity medicament.

9. Use of a composition according to any of claims 1 to 6 for the preparation of a pharmaceutical composition for the treatment and/or prevention of Diabetes mellitus or Obesity, by simultaneous, separate or sequential administration.

10. Use according to claim 9, wherein the pharmaceutical composition is for treatment and/or prevention of insulin resistant states of Diabetes.

11. Use according to claim 10, wherein the insulin-dependent state of Diabetes is Diabetes type 1 or a disease related thereto.

12. Use according to claim 10, wherein the insulin resistant state of Diabetes is Diabetes type 2 or a disease related thereto.

13. Kit for simultaneous, separate or sequential administration, comprising a pharmaceutically acceptable compound derived from the VIa and VIb groups of the periodic table and a pharmaceutically acceptable amine selected from the group consisting of semicarbazide-sensitive amine oxidase (SSAO) substrates, or a pharmaceutically acceptable salt of said amine, in admixture with pharmaceutically acceptable excipients or carriers.

14. Kit according to claim 13, wherein the kit is for treatment and/or prevention of insulin resistant states of Diabetes, preferably Diabetes type 2 or a disease related thereto, or for the treatment and/or prevention of obesity.
